# EUROPEAN PATENT APPLICATION

(11) **EP 3 649 941 A1**
(43) Date of publication of application: **13.05.2020**
(21) Application number: 18205696.0
(22) Date of filing: 12.11.2018
(51) Int. Cl.: A61B 5/145, A61B 5/00

(54) **DEVICE, SYSTEM AND METHOD FOR ESTIMATING AN ANALYTE CONCENTRATION IN SWEAT AS RELEASED BY A SWEAT GLAND**

(71) Applicant: Koninklijke Philips N.V., 5656 AG Eindhoven (NL)
(72) Inventor: JOHNSON, Mark Thomas, 5656 AE Eindhoven (NL); PELSSERS, Eduard Gerard Marie, 5656 AE Eindhoven (NL); VISWESWARA, Ashoka Sathanur, 5656 AE Eindhoven (NL); DELLIMORE, Kiran Hamilton J., 5656 AE Eindhoven (NL)
(74) Representative: de Haan, Poul Erik

(57) **Abstract**

The present invention relates to a device, system and method for estimating an analyte concentration in sweat as released by a sweat gland. The present invention further relates to a switching sensor device for sensing an analyte concentration in sweat as released by a sweat gland and to a trigger sensor device for sensing an analyte concentration in sweat as released by a sweat gland.

## Description

### FIELD OF THE INVENTION

The present invention relates to a device, system and method for estimating an analyte concentration in sweat as released by a sweat gland. The present invention further relates to a switching sensor device for sensing an analyte concentration in sweat as released by a sweat gland and to a trigger sensor device for sensing an analyte concentration in sweat as released by a sweat gland.

### BACKGROUND OF THE INVENTION

Sweat is a non-obtrusively accessible bio-fluid containing physiologically and metabolically rich information. In fact, sweat has many of the same analytes, i.e. biomarkers, chemicals and/or solutes, and analyte concentrations as found in blood and interstitial fluids. Sweat sensing is a non-invasive, continuous and prolonged monitoring of biomarkers, i.e. analytes, in sweat that can give information about health and/or well-being. There is therefore a demand to measure the most meaningful concentration of biomarkers in sweat, for example for monitoring dehydration, stress, sleep, children's health and in perioperative monitoring. This most meaningful concentration is the one existing when the sweat is produced by the sweat glands, preferably directly above the upper coiled duct on the skin.

Some examples of clinically relevant components of sweat are Na+, Cl- and/or K+ to monitor dehydration, lactate as early warning for inflammation, glucose for diabetics and neonates and cortisol for sleep and stress monitoring, for example.

The following table shows a non-exhaustive list of biomarkers known to be present in sweat, underlining the promise and relevance of sweat sensing to a diversity of applications. In the table, the term LOD stands for required limit of detection.

| **marker** | **LOD** | **applications** | **status** |
|---|---|---|---|
| Na⁺, K⁺, Cl⁻ | 10 mM | dehydration monitoring; Cystic Fibrosis diagnosis; | CF diagnosis from sweat in NICU clinical practice |
| Lactate | 10 mM | pressure ischemia detection; post-operative monitoring | predictive ischemia marker shown; only temporal correlation with blood shown |
| urea | 1 mM | skin conditions; dehydration monitoring | hypothesis: urea content in sweat keeps skin in good condition |
| ethanol | 1 mM | alcohol use monitoring | on-skin monitoring used in practice |
| ammonium | 1 mM | metabolic disorders | absolute correlation to blood shown |
| glucose | 0.1 mM | diabetes | temporal correlation to blood shown |
| cortisol | 10⁻⁴ mM | stress monitoring | absolute correlation to blood shown |

Further biomarkers known to be present in apocrine sweat comprise proteins, in particular apocrine secretion odor-binding proteins 1 and 2 (ASOB1 and ASOB2), carbohydrate, ferric ions, lipids, steroids, sialomucin and cathelicidin, wherein proteins and sialomicin have been shown to be present in eccrine sweat as well.

The concept of using sweat for non-invasive access to biomarkers and solutes in blood is not new, with clinical work showing promising results as far back as the 1940s and 1950s. However, until now an impactful application of sweat analysis has been limited mainly to cystic fibrosis diagnostics, and testing for drugs of abuse and alcohol. This is due to the fact that using sweat as a clinical sample for sweat analysis requires a minimum quantity of sweat. Sweat sampling, however, is compromised by sample evaporation and lack of appropriate sampling devices and a proper normalization of a sampled volume.

Furthermore, since a part of the analytes in sweat is reabsorbed by the skin the concentration measured on the surface of the skin may not be meaningful enough for a good diagnosis. In fact, the development of reliable sweat sensing has been hampered by several issues: First of all, results from sweat sensing have been highly variable. Secondly, the correlation between blood and sweat values appears to be lacking for various biomarkers. Thirdly, the focus of sweat sensing has been on sensors, not on reliable and robust collection methods for the minute amounts produced.

### SUMMARY OF THE INVENTION

It is an object of the present invention to provide a device, system and method for estimating an analyte concentration in sweat as released by a sweat gland.

In a first aspect of the present invention a device for estimating an analyte concentration in sweat as released by a sweat gland is presented, the device comprising:
- a measurement input unit configured to obtain at least one of flow rate, pH value and rate of change of pH value of sweat on a user's skin,
- a sensor input unit configured to obtain the analyte concentration in the sweat on the user's skin, and
- a processing unit configured to correct the analyte concentration according to a correction model for estimating the analyte concentration as released by the sweat gland based on at least one of the flow rate, the pH value and the rate of change of the pH value of the sweat.

In a second aspect of the present invention a method for estimating an analyte concentration in sweat as released by a sweat gland is presented, the method comprising:
- obtaining at least one of flow rate, pH value and rate of change of pH value of sweat on a user's skin,
- obtaining the analyte concentration in the sweat on the user's skin, and
- correcting the sensed analyte concentration according to a correction model for estimating the analyte concentration as released by the sweat gland based on at least one of the flow rate, the pH value and the rate of change of the pH value of the sweat.

In yet a further aspect of the present invention, there are provided a corresponding system and a computer program which comprises program code means to carry out the steps of the method for estimating an analyte concentration in sweat as released by a sweat gland when said computer program is carried out on the computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed system, method, computer program and medium have similar and/or identical preferred embodiments as the claimed device, in particular as defined in the dependent claims and as disclosed herein.

The present invention is based on the idea to provide a device configured to determine the concentration of an analyte in sweat as provided immediately after its excretion by a user's sweat glands.

In current devices for analyte concentration sensing sweat sample collection and the presentation to respective sensors is not well controlled so that continuous reliable sensing over a long period of time is difficult. Furthermore, current devices are not designed to handle the tiny amounts of sweat that are produced under normal conditions, i.e. in the order of nano-liters per minute per sweat gland.

The proposed invention has the advantage to take into account the reabsorption of analytes from sweat as the sweat leaves the sweat duct. Accordingly, effects of physiological mechanisms causing incorrect measurements of analyte concentrations in sweat are reduced or even eliminated by the present invention.

In the invention proposed, analyte concentrations as sensed on a user's skin are corrected with respect to the flow rate at which said sweat has been released by the sweat glands and/or with respect to the pH value the respective sweat has and/or with respect to a rate of change of the sweat's pH value. In fact, the present invention exploits the fact, that analyte concentration, flow rate, pH value and rate of change of pH value are mutually related. The correction model used is based on said mutual relation and particularly takes effects of analyte reabsorption, particularly occurring along the entire sweat duct from the sweat gland until the pore, into account. In fact, the correction model is configured to take any of flow rate, pH value and rate of change of the pH value as well as analyte concentration obtained as input in order to correct the analyte concentration based on any of the flow rate, pH value and rate of change of the pH value.

As used herein, the flow rate may be the rate at which sweat is refreshed at a measuring unit and therefore old sweat is removed as new sweat arrives. Preferably, the flow rate, pH value and/or rate of change of pH value obtained by the measurement input unit correspond to sweat originating from the same body region as the analyte concentration obtained by the sensor input unit. This is due to the fact that the relationship between flow rate and analyte concentration and likewise the relationship between pH value (and rate of change of the pH value) and analyte concentration may differ among different body regions.

The correction model used for correcting the sensed analyte concentration may comprise the provision of predetermined flow rate values, or pH values and/or predetermined values corresponding to a rate of change of pH value in a first step, and, for each predetermined flow rate value, pH value and/or rate of change of pH value the provision of a predetermined concentration of an analyte of interest in a second step. In a third step, each predetermined concentration of a sensed analyte is associated with a predetermined estimated concentration of an analyte.

To be more specific, first, the flow rate as obtained by the measurement input unit may be compared with predetermined flow rate values, and a predetermined flow rate value may be then selected from this comparison accordingly. To the predetermined flow rate values there may be associated a predetermined concentrations of an analyte, preferably in a one-to-one relationship. The analyte concentration obtained by the sensor input unit may then be compared with the predetermined concentrations of said analyte, and a predetermined concentration of analyte may be selected from this comparison. The estimated concentration of the analyte may be then the analyte concentration which is associated with the selected predetermined concentration of analyte. The same procedure may be applied to a pH value or a rate of change of a pH value measured, accordingly. The predetermined concentrations for analytes and their assignment to predetermined flow rates, for example, may be based on clinical measurement with reference to biofluids such as blood, for example.

However, the model may likewise be implemented to solely take into account the measured flow rate, pH value and/or rate of change of pH value. In fact, for a flow rate,pH value and/or rate of change of a pH value obtained there may be provided a predetermined analyte concentration, wherein the sensed analyte concentration may be replaced by said predetermined analyte concentration by the processing unit. This procedure may be applied to all flow rates, pH values and/or rate of change of pH values obtained or only to a selection of said values.

Furthermore the model may correct the obtained analyte concentration taking into account both the flow rate, pH value and/or rate of change of pH value obtained and the analyte concentration obtained. For example, given a pH value, said pH value may be compared with predetermined pH values and a predetermined pH coming closest to said obtained pH value may be selected. Likewise, the obtained concentration of an analyte may be compared with predetermined analyte concentrations and a predetermined analyte concentration coming closest to said obtained analyte concentration may be selected. Taking into account both the predetermined pH value and the predetermined analyte concentration the correction model may provide an estimated analyte concentration reflecting the true analyte concentration. This procedure may apply to a flow rate or rate of change of pH obtained accordingly.

By providing a correction model the concentration of an analyte as obtained by the sensor input unit may be corrected for reabsorption effects related to the sweat's flow rate and/or pH value and/or rate of change of pH. Obviously, if only a flow rate is obtained by the measurement input unit the correction for estimating the analyte concentration as released by a sweat gland may not take into account the pH value or the rate of change of a pH value, but only the flow rate. The same applies to a pH value and a rate of change of a pH value obtained. Furthermore, if the measurement input unit is configured to obtain both the flow rate and the pH value, the correction model may take into account either the flow rate or the pH value or both of them.

The present invention may be applied for non-invasive, semi-continuous and prolonged monitoring of analytes in sweat to indicate health status and well-being. In particular, the present invention may be applied for monitoring dehydration, stress, sleep, children's health and in perioperative monitoring, but can also be used for spot-check measurements. For example, in the field of patient monitoring the present invention may be used to gather an early warning for sudden deterioration of patients in the general ward and for investigation of sleep disorders, which currently only done in spot-check fashion when a patient is visiting a doctor. The present invention may be implemented in a patch, a wearable device like a smartwatch, for example, and the like.

In an embodiment of the device, the processing unit is configured to correct the sensed analyte concentration if at least one of the following conditions are met: the flow rate is below a first flow rate threshold, the pH value is below a first pH value threshold, the rate of change of the pH value is above a first rate of change threshold. Preferably, the first flow rate threshold, the first pH value threshold and/or the first rate of change threshold defines a point at which reabsorption of analytes reaches a saturation level. In general, the reabsorption rate of analytes decreases at higher flow rate and/or higher pH value and/or lower rate of change of the pH value. In fact, an increasing flow rate, i.e. an increase in sweating, goes along with an increase of the sweat's pH value. Furthermore, a higher flow rate suppresses the reabsorption of analytes, i.e. biomarkers, by the user's skin before sensing. In other words, when the rate of sweat reabsorption is higher, the sweat rate is lower and the pH is more acidic. As the rate of reabsorption decreases due at higher sweat rates, the pH becomes more neutral (and particularly close to pH 7). Therefore, the change of analyte concentration is lower above the saturation point of reabsorption. In particular, once the point of reabsorption saturation has been reached, the analyte concentration increases which allows for more precise results of measurements with respect to the analyte concentration. In fact, this ensures that the analyte concentration as measured for a flow rate, pH value and/or rate of change of pH value above said saturation threshold can be assumed to be equal to the true analyte concentration as secreted by the coiled tubular part of the glands because reabsorption effects become negligible above said threshold.

While the first flow rate threshold, the first pH value threshold and/or the first rate of change threshold of the pH value may correspond to the saturation of analyte reabsorption, these threshold may likewise be defined in another way. For example, the first flow rate threshold and/or the first pH value threshold may be defined to be above the saturation of analyte reabsorption to make sure that reabsorption effects are indeed negligible.

In general, it is assumed that with increasing flow rate and/or pH value and/or decreasing rate of change of pH value, respectively, the measured biomarker level, i.e. analyte concentration, becomes closer to the actual level as released by the glands and, in fact, more closely reflects the concentrations present in the user's blood. However, below the first flow rate threshold and/or the first pH value threshold mentioned above a correction of the sensed analyte concentration may be necessary due to physiological reabsorption mechanisms. The same may apply above the first rate of change threshold. Any of the first thresholds mentioned above may be predefined, particularly with respect to simulations or clinical tests, for example.

In another embodiment of the device, the first threshold for the flow rate ranges between 0.5 mg/min/cm² and 1.2 mg/min/cm², particularly between 0.8 mg/min/cm² and 1 mg/min/cm², and/or the first threshold for the pH value ranges between 2.5 and 6, particularly between 3 and 5, more particularly between 3.5 and 4.5 and/or the first threshold for the rate of change of the pH value ranges between 0.07 per minute and 0.12 per minute, particularly between 0.09 per minute and 0.11 per minute. Preferably, the first rate of change threshold for the rate of change of the pH value is at 0.1 per minute. A rate of pH change > 0.1 per minute may correspond to a high rate of reabsorption. A rate < 0.1 per minute may be associated with a low rate of reabsorption.

In fact, the threshold for saturation of sweat analytes has been measured to be at a flow rate of around 0.9 mg/min/cm² or at a pH value of around 4.

Preferably, any of said thresholds correspond to the point of saturation of analyte reabsorption.

In another embodiment, the correction model of the device for estimating an analyte concentration in sweat as released by a sweat gland comprises any of a look-up table, a graph, a mathematic function and an algorithm. For example, predetermined flow rate and/or pH values, the obtained measured values are compared with, may be provided in a look-up table. In other words, there may be provided a look-up table comprising a (true) concentration of a particular analyte of interest versus the sweat rate, wherein the relation between an analyte concentration as released by the sweat glands and sweat rate may be determined from a set of volunteers. Hence at each sweat rate value a correction factor between an analyte concentration as sensed in sweat and the (true) value in sweat without reabsorption is known. Likewise, predetermined analyte concentrations corresponding to obtained measured flow rates below a particular first flow rate threshold, for example, may be provided by a mathematic formula using the obtained flow rates as input parameters.

In a further embodiment of the device for estimating an analyte concentration in sweat as released by a sweat gland, the device further comprises a measuring unit configured to measure at least one of the flow rate, the pH value and the rate of change of the pH value of sweat on the user's skin. The measuring unit preferably comprises an electrical sensor, but may also comprise optical, chemical, mechanical or other known bio-sensing mechanisms. Flow rate can also in part be determined from solute generation, transport, advective transport of fluid, diffusion of transport of solutes or other factors that impact the flow rate. In particular, the measuring unit may configured to measure a flow rate by comparing a level of filling of sweat in a sweat collecting channel or tube at two different points in time, wherein the level of filling may be detected by a camera, for example. The measuring unit may be configured to measure the flow rate, pH value and/or rate of change of the pH value continuously or periodically.

In still a further embodiment, the device for estimating an analyte concentration in sweat as released by a sweat gland comprises a sensor unit configured to sense the analyte concentration in the sweat on the user's skin. The sensor unit may comprise any of an electrochemical sensor or other sensor type based on coloration, electrical impedance and/or labelled antibodies (for sensor types see, for example, Rathee et all, Biosensors based on electrochemical lactate detection: A comprehensive review, Biochemistry and Biophysics Reports 5 (2016) 35-54 and Bandodkar et all, Non-invasive wearable electrochemical sensors: a review, Trends in Biotechnology July 2014, Vol. 32 No. 7). The sensor unit may be configured to sense the analyte concentration continuously or periodically.

In another embodiment, the device further comprises a fluid switch configured to direct the sweat away from the sensor unit if at least one of the following conditions are met: the flow rate is below a second flow rate threshold, the pH value is below a second pH value threshold, the rate of change of the pH value is above a second rate of change threshold.

As used herein, directing the sweat away from the sensor unit comprises not directing the sweat to the sensor unit, i.e. preventing the sweat from being sensed by the sensor unit. The fluid switch may comprise any of a sweat impermeable valve, a sweat impermeable slide, a fabric configured to dissipate above a predefined pH value and/or flow rate and/or rate of change of pH value of sweat. The fluid switch may comprise polydimethylsiloxane, for example. Generally, the fluid switch can be based on various principles, for example an electro active polymer that can be activated electrically, a swelling hydrogel etc.

The fluid switch may likewise be configured to direct the sweat to the sensor unit if at least one of the following conditions are met: the flow rate is above a third flow rate threshold, the pH value is above a third pH value threshold, the rate of change of the pH value is below a third rate of change threshold.

In particular, the fluid switch may be configured to open and/or close an inlet configured to let pass the sweat to the sensor unit. Given a fluid switch which directs away the sweat from the sensor unit, said unit is not exposed to sweat. This is of particular advantage in situations where the sensor device is attached to the user's skin without any measurements being performed. In such cases the sensor unit is spared and not saturated. Accordingly, the lifetime of the sensor unit and the correction sensor device, respectively, is increased.

Preferably, if the flow rate and/or the pH value exceed(s) a predefined third flow rate threshold and/or a predefined third pH value threshold, respectively, and/or if the rate of change of the pH value is below a predefined third rate of change threshold, the fluid switch may be configured to direct the sweat towards the sensor unit whereby a sufficiently accurate measure of the concentration in the secreted sweat is measured, i.e. sensed, without unnecessarily saturating the sensor unit. In other words, the fluid switch may be configured to ensure that ample sweat is provided to the sensor unit such that reabsorption effects of analytes by the skin become negligible. Accordingly, the fluid switch is configured to ensure that the analyte concentration as sensed closely resembles the analyte concentration as released by the sweat glands.

The second and/or third flow rate threshold for the fluid switch may range between 0.5 mg/min/cm² and 1.2 mg/min/cm², particularly between 0.8 mg/min/cm² and 1 mg/min/cm², the second and/or third pH value threshold may range between 2.5 and 6, particularly between 3 and 5, more particularly between 3.5 and 4.5 and the second and/or third rate of change threshold may range between 0.09 per minute and 0.11 per minute and may particularly be at 0.1 per minute. The second and third thresholds for the flow rate, pH value and/or rate of change of the pH value may be the same or may be different, respectively. Furthermore, said values may be predefined based on a (clinical) test or simulation, for example.

In yet another embodiment of the device, the device further comprises a trigger unit configured to power the sensor unit and/or to trigger the fluid switch according to an exposure of the trigger unit to the sweat.

The trigger unit may be configured to react upon exposure to sweat, wherein said reaction may be of any of a mechanical, chemical, electrical and/or electrochemical reaction. In particular, the trigger unit may be connected to the measuring unit and obtain a flow rate, pH value and/or rate of change of pH value of sweat as measured on a user's skin. According to the flow rate, pH value and/or pH change value obtained, the trigger unit may be configured to power the sensor unit and/or to trigger the fluid switch.

The trigger unit may particularly power the sensor unit and/or trigger the fluid switch if a (predefined) threshold for said values is exceeded. In particular, the trigger unit may be configured to power the sensor unit and/or to trigger the fluid switch if at least one of the following conditions are met: the flow rate is above a fourth flow rate threshold, the pH value is above a fourth pH value threshold, the rate of change of the pH value is below a fourth rate of change threshold. For example, if the flow rate is (measured to be) higher than 0.9 mg/min/cm² the trigger unit may be configured to trigger the fluid switch. However, the trigger unit may likewise be configured to trigger the fluid switch at or above a predefined rate of change of pH, wherein the rate of change of pH may be determined by the measuring unit and/or the processing unit, for example. If triggered, the fluid switch may open an inlet for directing sweat to the sensor unit. Therefore, the trigger unit may be used to save electricity, i.e. to power the sensor unit only if necessary. Furthermore, the trigger unit may help spare the sensor unit by triggering, e.g. activating and/or deactivating and/or moving, the fluid switch according to usability. For example, if the pH value of sweat is lower than 1 the sensor unit may not be usable since an analyte concentration sensed at this pH value is highly prone to error.

In a preferred embodiment of the device for estimating an analyte concentration in sweat as released by a sweat gland, the trigger unit comprises a swelling element configured to swell according to exposure to the sweat, wherein the sensor unit is powered if a size of the swelling element is above a first power threshold and/or wherein the fluid switch is triggered if a size of the swelling element is above a first trigger threshold.

Vice versa, the swelling element of the trigger unit may be configured to shrink in response to an exposure to the sweat. In particular, the sensor unit may be unpowered (i.e. switched off) if a size of the swelling element is equal or below a second power threshold and/or the fluid switch may be triggered if a size of the swelling element is equal or below a second trigger threshold.

Generally, the first power threshold and the second power threshold may be the same or different. The same applies to the first and second trigger threshold. Furthermore, any of these thresholds may be predetermined based on prior tests, for example.

The swelling element of the trigger unit may comprise an environmentally responsive biomaterial swelling in response to any of sweat flow rate, pH value, rate of change of pH value, and skin temperature. The swelling element may comprise any of a poly methacrylic acid-co-acrylamide (poly mAA-co-AAm), combo-type grafted poly(N-isopropylacrylaminde)(nIPAAm), poly(acrylamide-acrylic acid) (pAM-AA) and poly(n-isopropylacrylamide-co-acrylamide) [p(nIPAAm-AM], wherein combo-type grafted poly(N-isopropylacrylaminde)(nIPAAm) is configured to swell due to moisture and wherein poly(n-isopropylacrylamide-co-acrylamide) [p(nIPAAm-AM)] is temperature sensitive. In particular, the swelling element may comprise poly(acrylamide-acrylic acid) (pAM-AA) super porous hydrogels (SPHs) which are pH sensitive and fast swelling.

The swelling element may be arranged as any of a pad, a patch, a layer and a strip. Swelling comprises an increase in thickness, volume and/or any other dimension of the element.

Furthermore, the trigger unit may be configured to comprise a conductive element. The conductive element may comprise any of a deflectable conductive membrane comprising, for example polydimethylsiloxane. In general, the conductive element may comprise any conductive polymer and may particularly comprise polyacetylene, polypyrrole, polyindole and polyaniline, also poly(p-phenylene vinylene). In fact, the swelling element may be the same as the conductive element. In this case the swelling element may comprise a conductive hydrogel, in particular a doped poly (N-acryloyl glycinamide-co-2-acrylamide-2-methylpropanesulfonic) (PNAGA-PAMPS) hydrogel with PEDOT/PSS, for example.

In general, the response time of the sensor unit, i.e. the time for the sensor unit to be powered, and/or the response time of the fluid switch to be triggered with respect to changes in the flow rate and/or pH value may depend on the rate of swelling of the swelling element. The response time may be reduced by using faster swelling elements, by miniaturization and/or by using a very thin hydrogel layer placed closely to the fluid switch.

In an embodiment of the device for estimating an analyte concentration in sweat as released by a sweat gland the measuring unit comprises any of a skin impedance sensor, CL- sensor, lactate sensor and electromechanical sensor and/or the sensor unit (16) comprises any of an electrochemical sensor, a coloration based sensor, an electrical impedance sensor and a sensor based on labeled antibodies.

In an embodiment of the device for estimating an analyte concentration in sweat as released by a sweat gland, the processing unit is configured to control the fluid switch.

In particular, the processing unit may be configured to control the fluid switch according to the second flow rate threshold, the second pH value threshold and/or the second rate of change threshold. More particularly, the fluid switch may be controlled to be in a closed if at least one of the following conditions are met: the flow rate is below a second flow rate threshold, the pH value is below a second pH value threshold, the rate of change of the pH value is above a second rate of change, wherein in the closed position the fluid switch is configured to direct sweat away from the sensor unit. On the other hand, for a flow rate, pH value and/or rate of change of pH value equal or above said respective thresholds (or the corresponding third thresholds) the fluid switch may be controlled to be in an open position, wherein in the open position the fluid switch is configured to let pass the sweat to the sensor unit. However, the processing unit may likewise control the fluid switch according to the respective first threshold(s).

In another embodiment of the device for estimating an analyte concentration in sweat as released by a sweat gland, the processing unit is configured to control the sensor unit. In particular, the processing unit may be configured to switch on and/or off the sensor unit.

In a further aspect of the present invention a switching sensor device for sensing an analyte concentration in sweat as released by a sweat gland is presented, the switching sensor device comprising:
- a sensor unit configured to sense on a user's skin the analyte concentration in the sweat as exposed to the sensor unit, and
- a fluid switch configured to direct the sweat on the user's skin away from the sensor unit if at least one of the following conditions are met:
   the flow rate is below a fifth flow rate threshold, the pH value is below a fifth pH value threshold, the rate of change of the pH value is above a fifth rate of change threshold.

In another aspect of the present invention a fluid switch method for sensing an analyte concentration in sweat as released by a sweat gland is presented, the fluid switch method comprising:
- sensing on a user's skin the analyte concentration in the sweat, and
- directing the sweat on the user's skin away from a sensor unit if at least one of the following conditions are met: the flow rate is below a fifth flow rate threshold, the pH value is below a fifth pH value threshold, the rate of change of the pH value is above a fifth rate of change threshold.

In still another aspect of the present invention a switching sensor system for sensing an analyte concentration in sweat as released by a sweat gland is presented, the switching sensor system comprising:
- a measuring unit configured to measure at least one of flow rate, pH value and rate of change of pH value of sweat on a user's skin,
- a measurement input unit configured to obtain at least one of the flow rate, the pH value and the rate of change of the pH value of sweat on a user's skin,
- a sensor input unit configured to obtain the analyte concentration in the sweat on the user's skin, and
- a processing unit configured to correct the analyte concentration according to a correction model for estimating the analyte concentration as released by the sweat gland based on at least one of the flow rate, the pH value and the rate of change of the pH value of the sweat, and
- a switching sensor device for sensing an analyte concentration in sweat as released by a sweat gland.

In yet a further aspect of the present invention, there is provided a corresponding computer program which comprises program code means to carry out the steps of the fluid switch method for sensing an analyte concentration in sweat as released by a sweat gland when said computer program is carried out on the computer as well as a non-transitory computer-readable recording medium that stores therein a computer program product, which, when executed by a processor, causes the method disclosed herein to be performed.

As used herein, directing sweat away from a sensor unit comprises not directing the sweat to a sensor unit, i.e. preventing the sweat from being sensed by a sensor unit. Furthermore the fluid switch may be configured to direct the sweat on the user's skin to a sensor unit if at least one of the following conditions are met: the flow rate is above a sixth flow rate threshold, the pH value is above a sixth pH value threshold, the rate of change of the pH value is below a sixth rate of change threshold. Preferably, the fifth flow rate threshold and the sixth flow rate threshold are the same. The same applies to the fifth and sixth pH value threshold and the fifth and sixth rate of change threshold.

Current devices used for analyte concentration sensing in sweat have the disadvantage that the devices are continuously monitoring sweat despite the fact that many measurements (particularly at lower sweat rate) are far from the actual secreted concentration. Such measurements may be prone to higher measurement errors and may indeed be superfluous as the analyte concentration at higher sweat rates are in any case more reliable. Furthermore, a continuous sensing may result in an earlier end-of life of an analyte concentration sensor as said sensor will become saturated more quickly. In addition in the device for estimating an analyte concentration in sweat as released by a sweat gland, the first measurement requires a semi-continuous probing of the look-up table which adds processing power requirements to the system. This is due to the fact that the analyte concentration as measured in the sweat and as excreted onto the skin depends on the secretion in the globular part and the reabsorption in the duct. Until the reabsorption is saturated the concentration will change due to the increasing secretion rate (i.e. flow rate) in the globular part of the gland. Hence there is required a correction mechanism, for example the mentioned look-up table.

The present invention is based on the idea of sensing an analyte concentration in sweat only in certain ranges of sweat production. Accordingly, the sensor unit may be preserved and the lifetime of the switching sensor device may be extended.

Embodiments of the switching sensor device may comprise any of the features claimed for the device for estimating an analyte concentration in sweat as released by a sweat gland as claimed in claims 1 to 6 and 8 to 10 and as described above.

In particular, any of the fifth flow rate threshold, the fifth pH value threshold and the fifth rate of change threshold defined above with respect to the fluid switch for directing the sweat on the user's skin away from the sensor unit may be the same as the second respective threshold(s) defined for the processing unit to correct the sensed analyte concentration of the device for estimating an analyte concentration in sweat as released by a sweat gland. However, any of the respective fifth and second thresholds may likewise be different.

Embodiments of the switching sensor device comprising a measurement input unit, sensor input unit and a processing unit may be used for calibration of the switching sensor device. In fact, a calibration of the switching sensor device may be achieved by correlating the concentration of an analyte in sweat at a given moment in time with the analyte concentration in a reference biofluid, such as blood or urine, drawn at the same time. This could be done in a hospital setting in which blood and/or urine samples are routinely taken during daily nursing rounds, for example.

In an embodiment of the switching sensor device, the processing unit may be configured to control the fluid switch. In particular, the processing unit may be configured to control the fluid switch according to the flow rate, pH value and/or rate of change of the pH value of the sweat. More particularly, the fluid switch may be controlled to be in a closed position if at least one of the following conditions are met: the flow rate is below the fifth flow rate threshold, the pH value is below the fifth pH value threshold, the rate of change of the pH value is above the fifth rate of change threshold, wherein in the closed position the fluid switch may be configured to direct sweat away from the sensor unit. However, the same may apply for the first flow rate threshold, the first pH value threshold and/or the first rate of change threshold. On the other hand, for a flow rate, pH value and/or rate of change of pH value equal or above said predefined threshold(s) the fluid switch may be controlled to be in an open position, wherein in the open position the fluid switch is configured to let pass the sweat to the sensor unit.

In another embodiment of the switching sensor device, the processing unit is configured to control the sensor unit. In particular, the processing unit may be configured to switch on and/or off the sensor unit.

In still a further aspect of the present invention a trigger sensor device for sensing an analyte concentration in sweat as released by a sweat gland is presented, the trigger sensor device comprising:
- a sensor unit configured to sense on a user's skin the analyte concentration in the sweat, and
- a trigger unit configured to power the sensor unit according to an exposure of the trigger unit to the sweat.

In another aspect of the present invention a trigger method for sensing an analyte concentration in sweat as released by a sweat gland is presented, the trigger method comprising:
- sensing on a user's skin the analyte concentration in the sweat, and
- powering a sensor unit according to an exposure of the trigger unit to the sweat.

In still another aspect of the present invention a trigger sensor system for sensing an analyte concentration in sweat as released by a sweat gland is presented, the trigger sensor system comprising:
- a measuring unit configured to measure at least one of flow rate, pH value and rate of change of pH value of sweat on a user's skin,
- a measurement input unit configured to obtain at least one of the flow rate, the pH value and the rate of change of the pH value of sweat on a user's skin,
- a sensor input unit configured to obtain the analyte concentration in the sweat on the user's skin, and
- a processing unit configured to correct the analyte concentration according to a correction model for estimating the analyte concentration as released by the sweat gland based on at least one of the flow rate, the pH value and the rate of change of the pH value of the sweat, and
- a trigger sensor device for sensing an analyte concentration in sweat as released by a sweat gland.

In yet a further aspect of the present invention, there is provided a corresponding computer program which comprises program code means to carry out the steps of the trigger method for sensing an analyte concentration in sweat as released by a sweat gland when said computer program is carried out on the computer as well as a non-transitory computer-readable recording medium that stores therein a computer program product, which, when executed by a processor, causes the method disclosed herein to be performed.

Current sensor devices for sensing an analyte concentration in sweat still require a high consumption of power. This is due to the fact that the flow rate sensors and/or pH sensor are generally continuously on. Moreover, even if there exists a switch mechanism used to switch on an analyte concentration sensor only when said sensor may provide a meaningful result, the switching mechanisms used generally require electrical power as well. Accordingly, there is a need to reduce power consumption of analyte concentration sensor devices.

In the present invention it is proposed to use a passive means to measure the sweat flow rate (or change in flow rate) and to trigger the fluid switch to direct the sweat towards an analyte concentration sensor. This will extend the lifetime of the device significantly as the device will only be powered when the sweat rate is in a range suitable for a meaningful analyte concentration sensing. Furthermore, the power consumption of said device is highly reduced.

The present invention is based on the correlation between sweat rate and pH changes in sweat.

Embodiments of the trigger sensor device may comprise any of the features claimed for the device for estimating an analyte concentration in sweat as released by a sweat gland as claimed in claims 1 to 7 and 9 to 10 and as described above.

In particular, embodiments comprising a measurement input unit, sensor input unit and a processing unit may be used for calibration of the trigger sensor device. The calibration of the trigger sensor device may be achieved by correlating the concentration of an analyte in sweat at a given moment in time with the analyte concentration in a reference biofluid, such as blood or urine, drawn at the same time. This could be done in a hospital setting in which blood and/or urine samples are routinely taken during daily nursing rounds, for example.

Preferably, the predefined threshold for the fluid switch to direct the sweat on the user's skin away from the sensor unit (and/or to the sensor unit), the predefined threshold(s) defined for the processing unit to correct the sensed analyte concentration of the device for estimating an analyte concentration in sweat as released by a sweat gland and the predefined power threshold as well as the predefined trigger threshold defined with respect to the trigger sensor device are related to each other. In particular, any of the first flow rate threshold, the second flow rate threshold, the third flow rate threshold, the fourth flow rate threshold, the fifth flow rate threshold and the sixth flow rate threshold may the same. However, any of these thresholds may be different. Furthermore, any of these thresholds may be predefined, in particular with respect to prior measurements, statistics, clinical tests and/or simulations. The same applies to the pH value thresholds and the rate of change thresholds.

### BRIEF DESCRIPTION OF THE DRAWINGS

These and other aspects of the invention will be apparent from and elucidated with reference to the embodiments described hereinafter. In the following drawings:
Fig. 1 shows a schematic diagram of a first embodiment of a device for estimating an analyte concentration in sweat as released by a sweat gland according to the present invention,
Fig. 2 shows a schematic diagram of a second embodiment of a device for estimating an analyte concentration in sweat as released by a sweat gland according to the present invention,
Fig. 3 shows a schematic diagram of a third embodiment of a device for estimating an analyte concentration in sweat as released by a sweat gland according to the present invention,
Fig. 4 shows a top view of an exemplary implementation of an embodiment of a device for estimating an analyte concentration in sweat as released by a sweat gland according to the present invention,
Fig. 5 shows a cross sectional view of an exemplary implementation of an embodiment of a device for estimating an analyte concentration in sweat as released by a sweat gland according to the present invention,
Fig. 6 shows a schematic diagram of a first embodiment of a system for estimating an analyte concentration in sweat as released by a sweat gland according to the present invention,
Fig. 7 shows a schematic diagram of a second embodiment of a system for estimating an analyte concentration in sweat as released by a sweat gland according to the present invention,
Fig. 8 shows an exemplary embodiment of correction model according to the present invention,
Fig. 9 shows a schematic diagram of a first embodiment of a switching sensor device for sensing an analyte concentration in sweat as released by a sweat gland according to the present invention,
Fig. 10 shows a schematic diagram of a second embodiment of a switching sensor device for sensing an analyte concentration in sweat as released by a sweat gland according to the present invention,
Fig. 11 shows a cross sectional view of an exemplary implementation of an embodiment of a device for sensing an analyte concentration in sweat as released by a sweat gland according to the present invention,
Fig. 12 shows a schematic diagram of an embodiment of a trigger sensor device for sensing an analyte concentration in sweat as released by a sweat gland according to the present invention,
Figs. 13A and 13B show a cross sectional view of an exemplary implementation of an embodiment of a trigger sensor device for sensing an analyte concentration in sweat as released by a sweat gland according to the present invention,
Fig. 14A and 14B show a cross sectional view of an exemplary implementation of an embodiment of a trigger device for sensing an analyte concentration in sweat as released by a sweat gland according to the present invention,
Fig. 15 shows the relationship between sweat rate and pH value of sweat for a duration of 60 minutes, respectively,
Fig. 16 shows the relationship between sweat sodium concentration and sweat rate for various body regions,
Fig. 17 shows a schematic relationship between analyte concentration in sweat and sweat rate as measured and as actually existing, and
Fig. 18 shows a relationship between the change in galvanic skin conductance and the sweat rate.

### DETAILED DESCRIPTION OF THE INVENTION

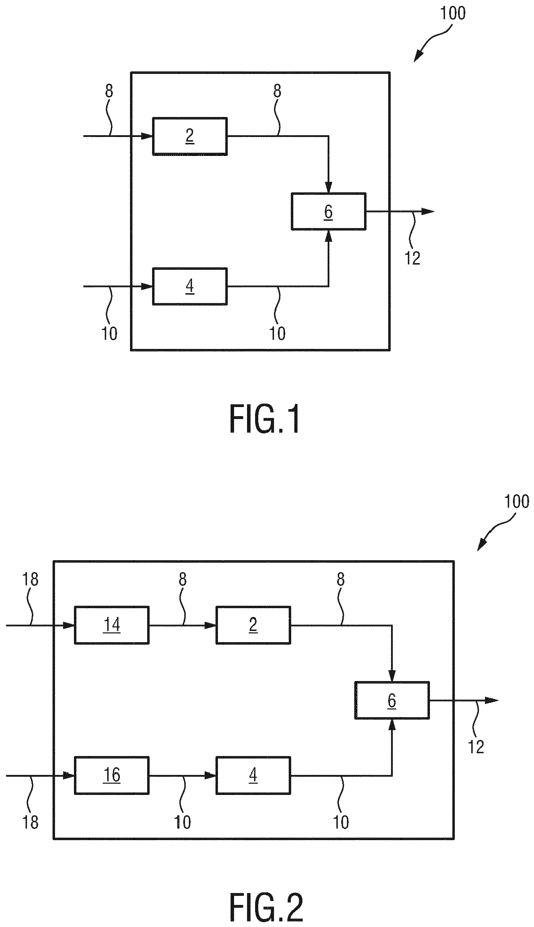

Fig. 1 shows a schematic diagram of a first embodiment of a device 100 for estimating an analyte concentration in sweat as released by a sweat gland according to the present invention. The device 100 comprises a measurement input unit 2, sensor input unit 4 and a processing unit 6.

In this embodiment, the measurement input unit 2 is configured to obtain a measurement result 8, for example a pH value of sweat on the user's skin. The sensor input unit 4 is configured to obtain an analyte concentration 10 in the user's sweat, wherein the pH value measurement and the analyte concentration preferably originate from the same body region of the user. In the processing unit 6 the analyte concentration 10 obtained by the sensor input unit 4 is corrected according to a correction model in order to more closely reflect the analyte concentration as released by the sweat glands. In particular, the correction model is configured to correct the analyte concentration obtained based on the obtained pH value. The processing unit 6 is further configured to provide the corrected analyte concentration as output 12.

Fig. 2 shows a schematic diagram of a second embodiment of a device 100 for estimating an analyte concentration in sweat as released by a sweat gland according to the present invention. The device 100 comprises measurement input unit 2, sensor input unit 4, processing unit 6, a measuring unit 14 and a sensor unit 16.

The measuring unit 14 is configured to obtain a sweat sample 18 from the user's skin and to measure the flow rate of sweat of said sample 18 on the user's skin and to provide the measurement result 8, i.e. in this embodiment the flow rate, to the measurement input unit 2, which is configured to provide the measurement result 8 to the processing unit 6. The sensor unit 16 likewise is configured to obtain said sweat sample 18. The sensor unit 16 is further configured to sense the analyte concentration 10 in the sweat and to provide the analyte concentration 10 to the sensor input unit 4. The processing unit 6 is configured to obtain both the flow rate and the analyte concentration from the measurement input unit 2 and the sensor input unit 4, respectively. The processing unit 6 further is configured to correct the sensed analyte concentration 10 for concentration changes of the sweat's analytes that have occurred between the sample's release from the sweat glands and the time of analyte concentration sensing. In order to correct said changes, which are predominated by re-absorption of analytes, the processing unit 6 makes use of the flow rate measured for the sample. In general, the higher the flow rate, the lower the re-absorption rate. Accordingly, the lower the flow rate, the stronger the corrections by the processing unit. In this embodiment, corrections are only performed for flow rates measured to be below 0.7 mg/min/cm². In particular, corrections are performed via a correction model, wherein for flow rates below 0.7 mg/min/cm² there is provided a corresponding predetermined analyte concentration which is assumed to represent the true analyte concentration. Accordingly, for flow rates below 0.7 mg/min/cm² the processing unit is configured to provide a predetermined analyte concentration as output 12, whereas for flow rates equal to or above 0.7 mg/min/cm² the processing unit provides the sensed analyte concentration output 12.

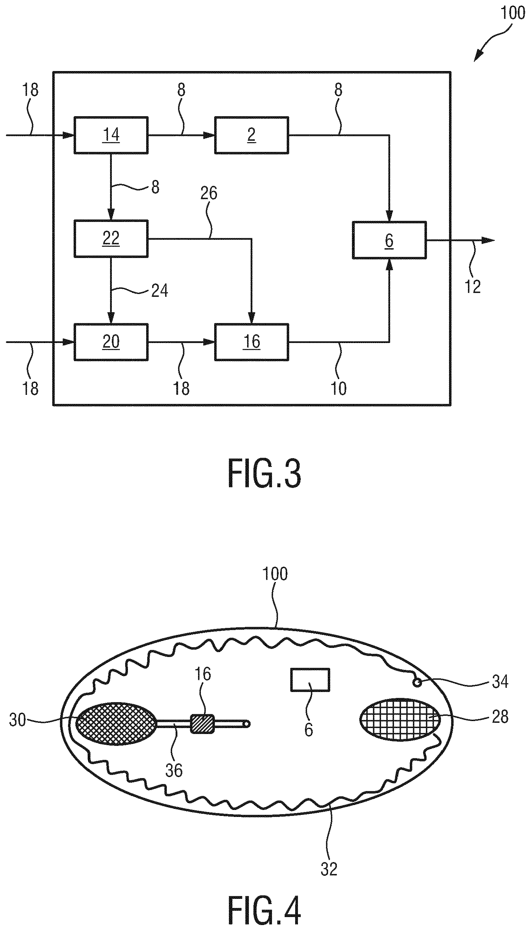

Fig. 3 shows a schematic diagram of a third embodiment of a device 100 for estimating an analyte concentration in sweat as released by a sweat gland according to the present invention. The device 100 comprises a measurement input unit 2, sensor input 4, a processing unit 6, a measuring unit 14, a sensor unit 16, a fluid switch 20 and a trigger unit 22.

After obtaining a sweat sample 18 from the user's skin the measuring unit 14 is configured to measure the pH value of said sample over time. In particular, the measuring unit 14 may be configured to measure the rate of change of the pH value over time. The measurement result 8 is then provided to the processing unit 6 (via the measurement input unit 2) and to the trigger unit 22. Depending on the measurement result 8, the trigger unit 22 may be configured to activate and/or deactivate the fluid switch 20 and the sensor unit 16. Accordingly, the trigger unit 22 is configured to provide a trigger signal 24 to the fluid switch 20, wherein the trigger signal 24 comprises information for the fluid switch 20 to open and/or close a sweat inlet of the device 100. The trigger unit 22 is further configured to provide a power signal 26 to the sensor unit 16, wherein the power signal 26 comprises information for the sensor unit to obtain electrical power or not. For example, for a rate of change of the pH value measured to be below 0.1 per minute, the trigger unit 22 may be configured to activate the fluid switch 20 and the sensor unit 16. Assuming a drop in sweat production, the trigger unit 22 may be configured to deactivate both the fluid switch 20 and the sensor unit 16 at a pH value rate of change equal or above 0.1 per minute. Although the thresholds for activation and deactivation of the fluid switch 20 and the sensor unit 16 have been chosen to be the same in this example, the fluid switch 20 may likewise be triggered at another rate of change threshold than the one used as threshold for activating the sensor unit 16. In case the fluid switch 20 is activated by the trigger unit 22, the fluid switch 20 is configured to direct the sweat, for which the pH value change has been measured by the measuring unit 14, towards the sensor unit 16. If the sensor unit 16 has also been activated by the trigger unit 22, the sensor unit 16 is configured to analyse the sweat directed to it with respect to a concentration of one or more analytes.

The results of said analysis are then provided to the processing unit 6, wherein the processing unit 6 is configured to correct the sensed one or more analyte concentrations 10 in order to provide one or more analyte concentrations reflecting the concentration of one more analytes as released by the sweat glands as output 12. In order to correct a sensed analyte concentration the processing unit 6 is configured to use a correction model taking into account the pH values over time, particularly the rate of change of the pH value of the sweat. The correction model in this embodiment comprises a look-up table wherein a rate of change of a pH value is associated with a predetermined reabsorbtion rate for an analyte of interest. Given the reabsorption rate and the sensed analyte concentration 10 the model is configured to estimate the true analyte concentration.

Fig. 4 shows a top view of an exemplary implementation of an embodiment of a device 100 for estimating an analyte concentration in sweat as released by a sweat gland according to the present invention. The device 100 is implemented as a patch configured to be attached on the user's skin. The patch comprises two sweat inlets 28 and 30 configured to collect sweat and provide the sweat to a measuring unit 14 and a sensor unit 16, respectively. The measuring unit 14 (not shown) is configured to measure the flow rate by determining the length of the filling of a thin channel 32 configured to transport the sweat collected by the sweat inlet 28. In particular, the flow rate may be determined by measuring difference in the lengths of fillings for two different points in time. Said difference may be determined, for example, by obtaining an image and determining the position of the meniscus in the channel 32 as a function of time. At the end of the cannel 32 there is arranged an air vent 34 for ventilating the channel 32. Simultaneous to the measurement of the flow rate of sweat, there is sensed a concentration of an analyte in the sweat by the sensor unit 16. Preferably, the sweat for the flow rate measurement and the sweat used to sense the analyte concentration 10 originate from the same body region of the user.

In this embodiment, the sensor unit 16 comprises an electrochemical base sensor configured to sense a sweat sodium concentration, for example. The sensor unit 16 is arranged at a thin tube 36 configured to transport the sweat collected by the sweat inlet 30 to the sensor unit 16. The sensor unit 16 may either be arranged inside the tube 36 or in a hole at the tube's wall. Both the results for the measurement of the flow rate and the sensed analyte concentration may be used as input for a look-up table stored in the processing unit 6. Using the look-up table the processing unit 6 may be configured to translate the observed concentration to an estimated originally secreted concentration.

Not depicted in Fig. 4 are the connections to and from the respective units. These connections may be wires configured to powering the units and/or wires for transmitting and/or receiving signals to and/or from the unit. In addition, the device 100 may comprise an onboard chip with an antenna configured to receive wireless power and/or to transmit and/or receive wireless signals to and/or from an instrument recording said signal and/or providing power.

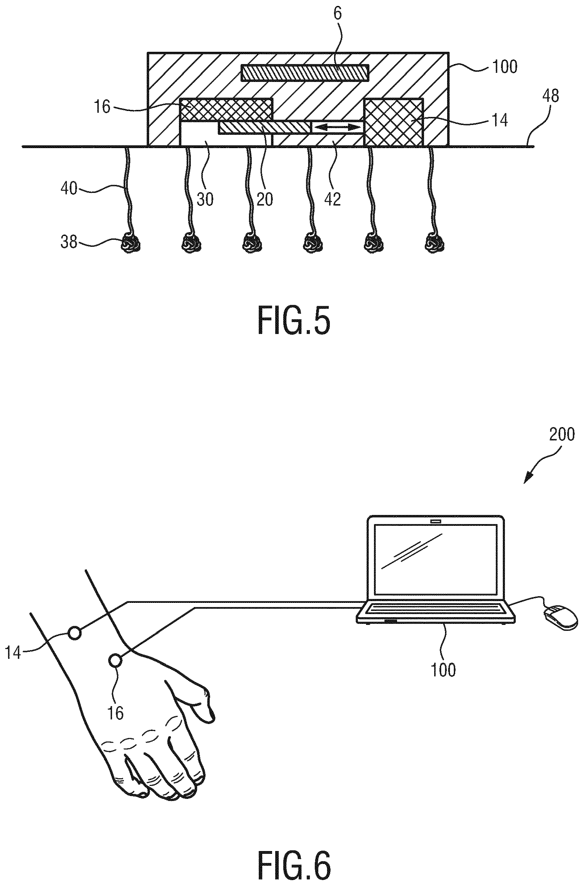

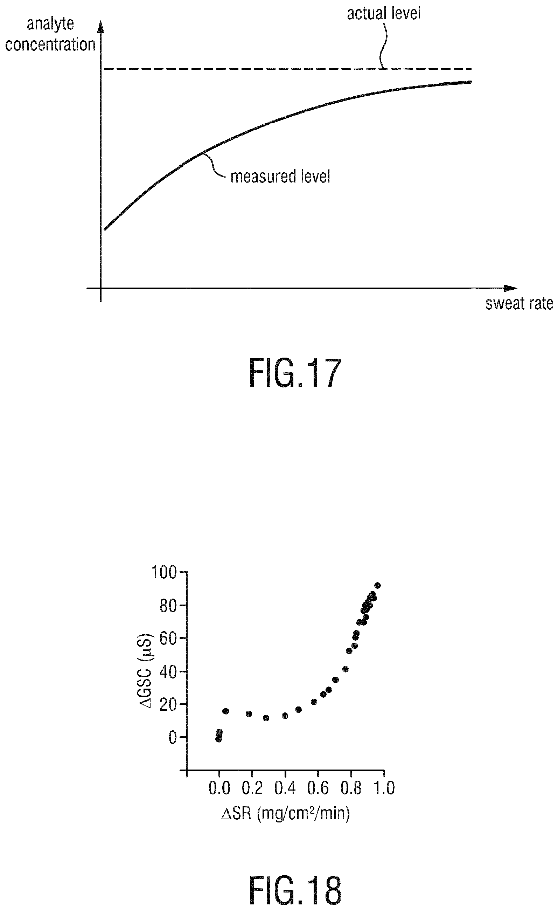

Fig. 5 shows a cross sectional view of an exemplary implementation of an embodiment of a device 100 for estimating an analyte concentration in sweat as released by a sweat gland according to the present invention. In this embodiment, the device 100 is implemented as wearable device, particularly as a patch. The device 100 is shown as attached to a user's skin 48, wherein there are furthermore depicted several sweat glands 38 and sweat ducts 40. In this embodiment, the device 100 comprises a measurement input unit 2, a sensor unit 4, a processing unit 6, a measuring unit 14, a sensor unit 16 and a fluid switch 20. The measuring unit 14 may be configured to continuously record the flow rate of the sweat. The measuring unit 14 in this embodiment is a galvanic skin conductance sensor, where the flow rate is essentially linear with the measured conductance between two electrodes positioned on the skin surface. However, the measuring unit 14 may comprise any other flow rate sensor such as a thermal flowmeter, for example. The measurement results of the measuring unit 14 are provided to the processing unit 6 of the device 100. The fluid switch 20 comprises a sweat impermeable slide configured to slide on the sweat (and particularly analyte) impermeable substrate 42 on the skin facing side of the device 100. In particular, the slide may slide between an open position and a closed positon, wherein in the open position the slide is turned back to open an inlet 30 configured to (let) pass sweat from the user's skin to the sensor unit 16. In this embodiment, the processing unit 6 is configured to control movement of the fluid switch 20. In particular, the processing unit 6 may cause the fluid switch 20 to be in the open or closed position. In fact, the processing unit 6 may control the position of the fluid switch 20 according to the measurement results of the flow rate. If the flow rate is below a predefined second flow rate threshold, the processing unit 6 may control the fluid switch 20 to be in the closed position, whereas for a flow rate equal or above said predefined second flow rate threshold, the processing unit 6 may control the fluid switch 20 to be in the open position. Accordingly, the fluid switch 20 may be configured to direct the sweat away from the sensor unit 16 if the flow rate is below said threshold. The predefined second flow rate threshold may range between 0.5 mg/min/cm² and 1.2 mg/min/cm², particularly between 0.8 mg/min/cm² and 1 mg/min/cm². However, said threshold may likewise be defined using the relationship shown in Fig. 18. For example, the second flow rate threshold may be defined as the point where a sensed concentration exceeds a certain percentage of the actual value according to Fig. 18, e.g. where a sensed concentration exceeds 75% or exceeds 90% of the actual value. Therefore, in this embodiment, only when the flow rate exceeds the predefined second flow rate threshold is the fluid switch 20 configured to direct the sweat towards the sensor unit 16 such that a sufficiently accurate measure of the concentration in the secreted sweat is measured without unnecessarily saturating the sensor unit 16. In this manner the sensor unit 16 is less exposed to sweat and the lifetime of the device 100 may increase.

In order to refine sensed analyte concentrations the processing unit may provide a look-up-table for correcting the sensed concentrations with respect to the flow rate.

However, instead of measuring the flow rate the measuring unit 14 may be configured to continuously record the pH value of the sweat, wherein the measurement results of the pH value may be provided to the processing unit 6 of the device 100 and wherein the processing unit 6 may control the position of the fluid switch 20 according to the measurement results of the pH value. For example, the processing unit 6 may only cause the fluid switch 20 to be in the open position if the pH value is above 4, more preferably above 4.5, even more preferably above 5.

Not depicted in Fig. 5 are the measurement input unit 2 and the sensor input unit 4. Furthermore, connections to and from the respective units are neither shown. These connections may be wires configured to power the units and/or wires for transmitting and/or receiving signals to and/or from the units. In addition, the device 100 may comprise an antenna configured to receive wireless power and/or to transmit and/or receive wireless signals to and/or from an instrument recording said signal and/or providing power.

Fig. 6 shows a schematic diagram of a first embodiment of a system 200 for estimating an analyte concentration in sweat as released by a sweat gland according to the present invention. The system 200 comprises a device 100 for estimating an analyte concentration in sweat as released by a sweat gland, a measuring unit 14 and a sensor unit 16, wherein the device 100 comprises at least a measurement input unit 2, a sensor input unit 4 and a processing unit 6. The measuring unit 14 and the sensor unit 16 may be comprised in adhesive patches attached to the skin of a user's wrist. The device 100 may be implemented in a laptop. The system 200 as illustrated in Fig. 6 may, e.g., be located in a hospital, medical practice, healthcare facility or the like.

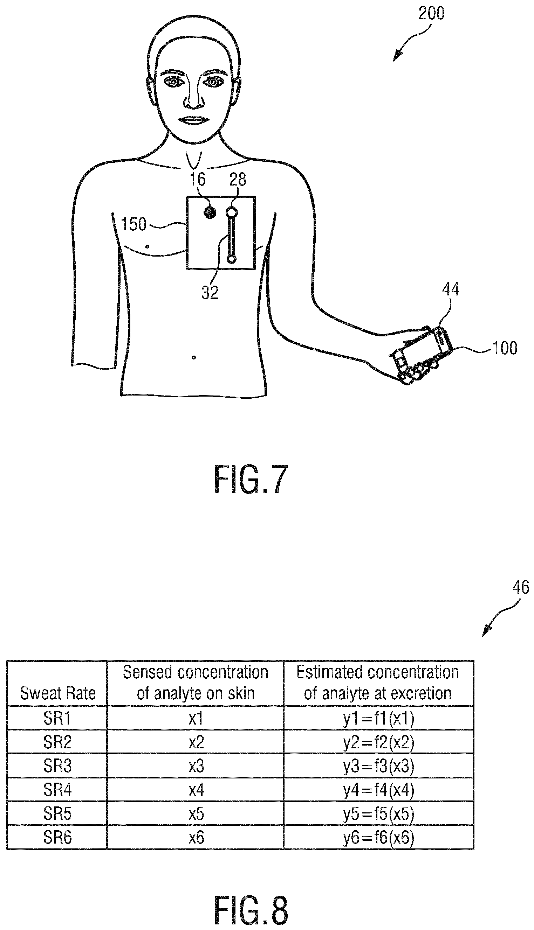

Fig. 7 shows a schematic diagram of a second embodiment of a system 200 for estimating an analyte concentration in sweat as released by a sweat gland according to the present invention. The system 200 comprises a device 100 for estimating an analyte concentration in sweat as released by a sweat gland and a sensor unit 16, a sweat inlet 28 and a thin channel 32 for transporting sweat away from the sweat inlet 28, wherein the sensor unit 16, the sweat inlet 28 and the thin cannel 32 are comprised in a sensor patch 150 attached to the user's skin. The device 100 is implemented in a smartphone in this embodiment and comprises a measurement input unit 2, a sensor input unit 4, processing unit 6, a measuring unit 14 and a camera 44. In this embodiment, the camera is configured to record a filling level of sweat in the thin channel 32 with respect to time. Using this information, the measuring unit 14 is configured to determine the flow rate. Furthermore, the sensor unit 16 is configured to sense an analyte concentration in the sweat of the user and to provide said concentration to the sensor input unit 4. In this embodiment, the sensor unit 16 may be configured to transmit the sensed analyte concentration to the sensor input unit 4 in a wireless manner.

Fig. 8 shows an exemplary embodiment of correction model 46 according to the present invention. In this embodiment, the correction model 46 comprises look-up table, wherein for each sweat rate, SR, a sensed concentration of an analyte in the sweat on a user's skin is assigned to an estimated concentration of said analyte at the location and time of excretion. The estimated concentration of the analyte is determined in this embodiment via a mathematical function of the sensed analyte concentration.

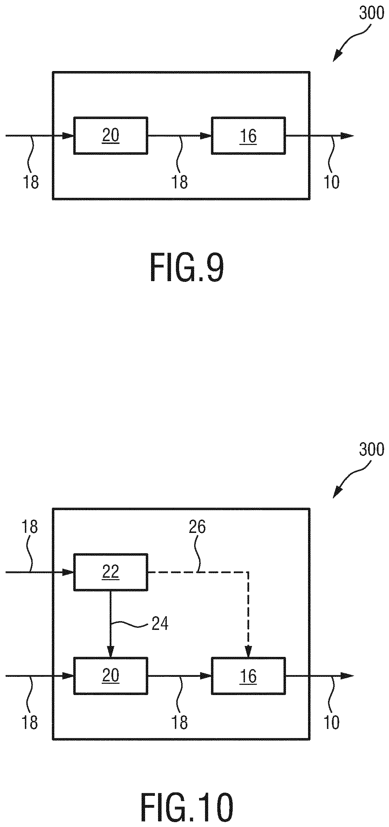

Fig. 9 shows a schematic diagram of a first embodiment of a switching sensor device 300 for sensing an analyte concentration in sweat as released by a sweat gland according to the present invention. In this embodiment, the device 300 comprises a sensor unit 16 and a fluid switch 20. The fluid switch 20 is configured to direct a sweat sample 18 on the user's skin away from the sensor unit if the pH value of the sweat is below a predefined fifth pH value threshold. However, the pH value of the sweat turns out to be equal or above said fifth pH value threshold the fluid switch 20 is configured to let the sweat of the sweat sample 18 pass to the sensor unit 16. The sensor unit 16 is configured to then sense the analyte concentration 10 in the sweat of said sample as exposed to the sensor unit 16.

Fig. 10 shows a schematic diagram of a second embodiment of a switching sensor device 300 for sensing an analyte concentration in sweat as released by a sweat gland according to the present invention. In this embodiment, the switching sensor device 300 comprises a sensor unit 16, a fluid switch 20 and a trigger unit 22. Depending on an exposure of the trigger unit 22 to sweat of a sweat sample 18 on a user's skin, the trigger unit 22 is configured to provide a trigger signal 24 to the fluid switch 20, wherein the fluid switch 20 may decide between directing said fluid sample away or directing said fluid sample to the sensor unit 16 in accordance with the trigger signal 24. Provided that the fluid switch 20 directs the sweat sample 18 to the sensor unit 16, the sensor unit 16 may sense a concentration of an analyte of interest in the sweat. Optionally, the trigger unit 22 may further be configured to provide a power signal 26 to the sensor unit 16, wherein the power signal 26 may depend on the exposure of the trigger unit 22 to sweat. Depending on the information comprised in the power signal 26 the sensor unit 16 may either be switched on or switched off. Preferably, the conditions for providing a trigger signal 24 configured to cause the fluid switch 20 to let the sweat pass to the sensor unit 16 are the same conditions for providing a power signal 26 configured to cause the sensor unit 16 to be switched on. The same applies analogously to permitting passage to the sensor unit 16 and switching off the sensor unit 16.

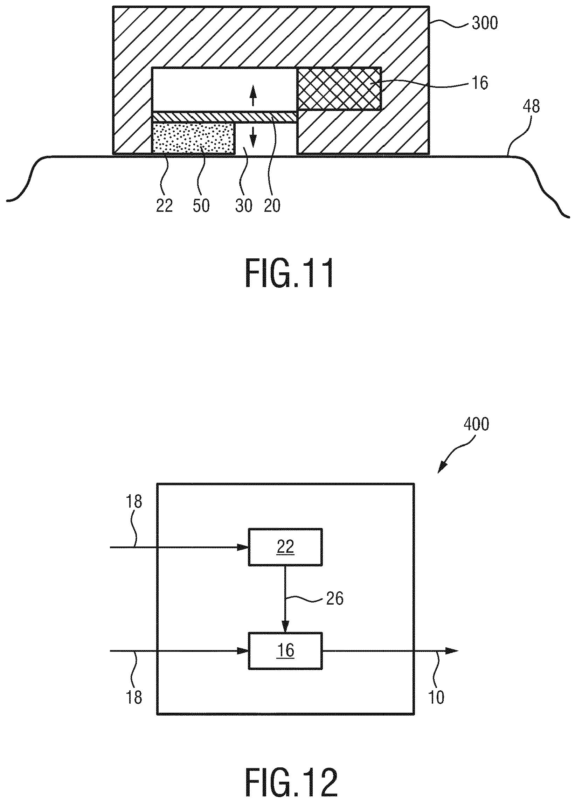

Fig. 11 shows a cross sectional view of an exemplary implementation of an embodiment of a device 300 for sensing an analyte concentration in sweat as released by a sweat gland according to the present invention. In Fig. 11 the device 300 is attached to the user's skin 48. The device 300 comprises a sensor unit 16, a fluid switch 20, a sweat inlet 30 and a trigger unit 22. The trigger unit 22 comprises a swelling element 50 configured to swell in accordance to an exposure to sweat on the user's skin. In particular, the swelling element 50 may comprise a pH responsive hydrogel, i.e. a hydrogel which is configured to swell in response to a change of a pH value it is exposed to. The swelling element 50 may be configured as a pad which swells in response to an increasing sweat pH with increasing sweat rate of the user. Vice versa, the swelling element 50 may be configured to shrink in response to a decreasing pH value. In this embodiment, there is a fluid switch 20 attached to the upper side of the trigger unit 22. The fluid switch 20 may comprise a sweat, and particularly analyte, impermeable material. For example, the fluid switch 20 may comprise a piece of plastic. The fluid switch 20 is configured to open and close the sweat inlet 30 of the device 300. In fact, in this embodiment, the fluid switch 20 is triggered by the trigger unit 50 to close an open the sweat inlet 30. Depending on a change in the pH value of the user's sweat the trigger unit 22 is configured to swell or shrink. In particular, the trigger unit 22 is configured to increase or decrease its size in direction of the fluid switch 20. Accordingly, the fluid switch 20 gets lifted or lowered by the trigger unit 22 in accordance with the pH value of the user's skin. If the fluid switch 20 gets lifted above a predefined first trigger threshold, sweat of the user's skin may pass through the sweat inlet 30 to the sensor unit 16. However, if said predefined threshold is not crossed, the fluid switch 20 is configured to prevent the sweat from passing through the sweat inlet 30 to the sensor unit 16. Accordingly, below a predefined pH value threshold the sensor unit 16 may not sense an analyte concentration in the user's sweat and may be spared for a meaningful analyte concentration sensing.

Fig. 12 shows a schematic diagram of an embodiment of a trigger sensor device 400 for sensing an analyte concentration in sweat as released by a sweat gland according to the present invention. In this embodiment, the trigger sensor device 400 comprises a sensor unit 16 and a trigger unit 22. Depending on an exposure of the trigger unit 22 to sweat of a sweat sample 18 the trigger unit 22 is configured to provide a power signal 26 to the sensor unit 16, wherein the power signal 26 is configured to cause the sensor unit 16 to be switched on or off, respectively. If the sensor unit 16 is switched on, the sensor unit 16 is configured to sense the analyte concentration 10 in the sweat sample as exposed to the sensor unit 16. The power signal 26 is not limited to an electromagnetic signal but may likewise comprise a physical change in the trigger unit. In fact, the power signal may comprise reaching a predefined size of the trigger unit.

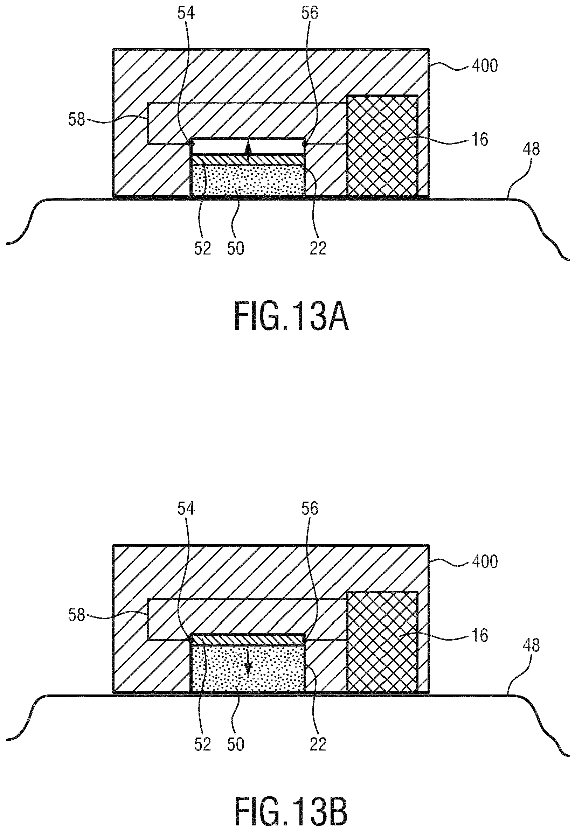

Figs. 13A and 13B show a cross sectional view of an exemplary implementation of an embodiment of a trigger sensor device 400 for sensing an analyte concentration in sweat as released by a sweat gland according to the present invention. In this embodiment, the device 400 comprises a sensor unit 16 and a trigger unit 22, wherein the sensor unit 16 is configured to be in direct contact with the user's skin 48 and wherein the trigger unit 22 comprises a swelling element 50 and a conductive element 52. The swelling element 44 may be configured to swell according to an exposure to sweat. In this embodiment, the conductive element 52 is implemented as a conductive layer attached to the swelling element 50, wherein the conductive layer is configured to be uplifted by the swelling element 50 towards two power connectors 54 and 56 when the swelling element 50 swells. In this embodiment, the swelling element 50 is configured to swell with an increased exposure of the swelling element 50 to sweat. Therefore, the more the user sweats, the more the trigger unit swells 22 and the higher is the conductive element 52 lifted. In fact, at a predefined sweat rate the conductive element 52 is lifted so high that the conductive element 52 comes into contact with the power connectors 54 and 56. If the conductive element 52 contacts the power connectors 54 and 56 a power circuit 58 is closed, wherein said power circuit 58 is configured to supply the sensor unit 16 with electrical power. Therefore, if the user's sweat rate reaches a predefined threshold, the sensor unit 16 may sense an analyte concentration in the user's sweat. Below said threshold the sensor unit 16 is switched off and may not sense any analyte concentration.

Fig. 13A shows the cross sectional view of the exemplary implementation of the embodiment of the trigger sensor device 400 with open power circuit 58. Fig. 13B shows the cross sectional view of the exemplary implementation of the embodiment of the device 400 with closed power circuit 58.

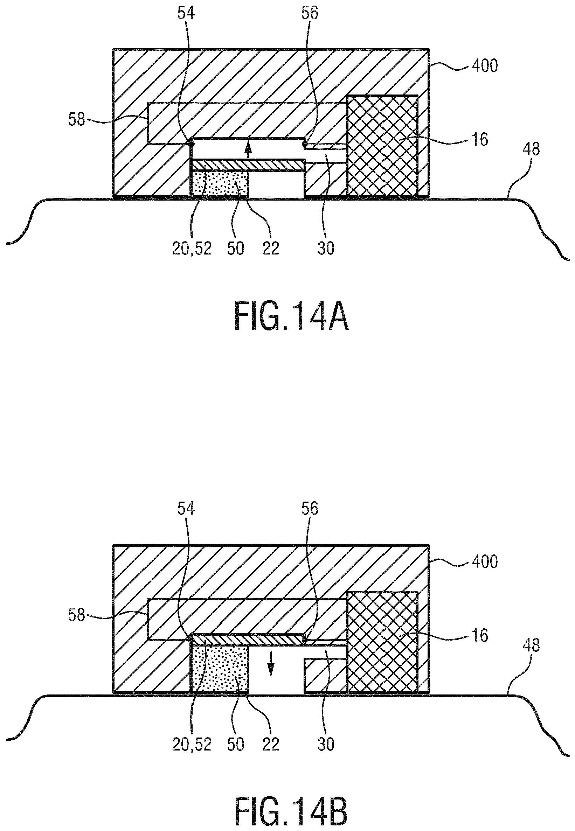

Fig. 14A and 14B show a cross sectional view of an exemplary implementation of an embodiment of a trigger device 400 for sensing an analyte concentration in sweat as released by a sweat gland according to the present invention. In this embodiment, the device 400 comprises a sensor unit 16, a fluid switch 20 and a trigger unit 22, wherein the trigger unit 22 comprises a swelling element 50 and a conductive element 52. In fact, in this embodiment, the conductive element 52 may comprise the fluid switch 20 and vice versa.

The functions of the trigger unit 22 and the fluid switch 20 are the same as in the embodiment of the trigger sensor device 400 shown in Figs. 13A and 13B. However, contrary to the embodiment of the trigger sensor device 400 shown in Figs. 13A and 13B, the embodiment shown in Figs. 14A and 14B further comprises a sweat inlet 30, wherein the fluid switch 20, i.e. the conductive element 52 in this embodiment, is configured to open and/or close the sweat inlet 30.

The trigger unit 22 comprises a swelling element 50 and is configured to trigger a fluid switch 20 in response to an exposure of the trigger unit 22 to a particular pH value or pH value range. The trigger unit 22 is further configured to close an electric circuit 58 for supplying the sensor unit 16 with electrical power. In this embodiment, the swelling element 50 is represented by a hydrogel layer configured to be attached to the user's skin. The layer may start swelling at a pH value of about 4, for example. While the lower side of the hydrogel layer is configured to face the user's skin 48, there is attached a conductive layer, i.e. a conductive element 52, on the upper side of the hydrogel layer. The swelling element 50 comprises a pH responsive hydrogel, i.e. a hydrogel which is configured to swell in response to a change of a pH value it is exposed to. In particular, the swelling element 50 is configured as a pad which swells in response to the increasing pH with increasing sweat rate. In this embodiment, the swelling element may comprise poly mAA-co-AAm, for example. The conductive element 52 may comprise a conductive membrane comprising polydimethylsiloxane, for example. If the trigger sensor device 400 is attached to the user's skin the hydrogel pad starts to swell as soon as the user starts to sweat enough. The more the user sweats, the more the pH value increases and the bigger becomes the pad in accordance. The amount of swelling depends on the increase of the pH value in the user's sweat. In fact, the elevation of the hydrogel pad in a direction away from the skin corresponds to a particular pH value. Since the conductive membrane is attached to the swelling element 50, also the membrane gets lifted upwards upon an increasing sweat rate. Once the conductive membrane reaches a particular height above the user's skin due to the swelling of the pad, the conductive membrane closes an electric circuit 58 which is configured to supply the sensor unit 16 with electrical power. In particular, the membrane may be configured to touch two electric conductors 54 and 56 at a predefined elevation of the swelling element 50 in order to close an electric circuit 58 between these conductors 54 and 56. At the same time, the membrane is lifted to a height where the sweat inlet 20 is opened. In other words, the trigger unit 22 of this embodiment is configured to both power the sensor unit 16 and trigger the fluid switch 20, wherein the power threshold and the trigger threshold coincide.

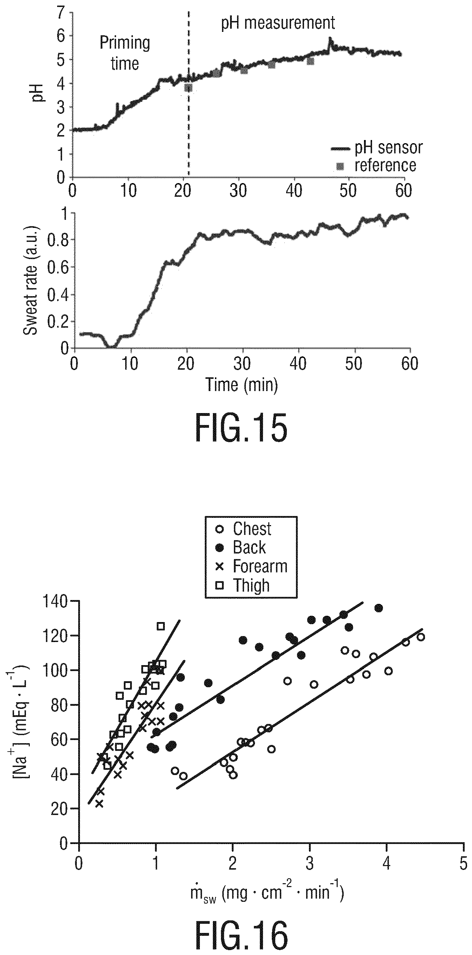

Fig. 15 shows the relationship between sweat rate and pH value of sweat for a duration of 60 minutes.

In Fig. 15 at the starting time the pH value is about 2 and the sweat rate is about 0.1 mg/min/cm². In the time between about seven minutes after start and 22 minutes after start the sweat rate increases significantly. 22 minutes after start the sweat rate is about 0.9 mg/min/cm². From this time on the sweat rate still increases, but only to about 1 mg/min/cm² at the end of measurement after 60 minutes. The pH value increases as the sweat rate increases. In fact, between minutes 7 and 22, the pH value increases at a higher rate than at the rest of the time. In fact, a high rate of change of pH occurs when the sweat reabsorption rate is high (i.e. at low pH values and sweat rates). At higher sweat rates the rate of reabsorption is decreased and this corresponds to a lower rate of change of pH. As can be seen from Fig. 15, the pH value changes from about 2 to about 4 in about 15 minutes from the start (in particular, before the dashed line indicating the start of measurement), while the pH value changes from about 4 to about 6 in about 35 minutes from the start.

The reference points indicated in Fig. 15 correspond to measurements taken with a reference patch at another location on the user's back.

Fig. 16 shows the relationship between sweat sodium concentration and sweat rate for various body regions. As can be seen the secretion rate of sweat (i.e. the sweat rate or flow rate) is proportional to the sweat sodium concentration in the sweat, wherein the proportionally depends on the body region.

Fig. 17 shows a schematic relationship between analyte concentration in sweat and sweat rate as measured and as actually existing. As can be seen from Fig. 17 the measured analyte concentration comes closer to the true analyte concentration the higher the sweat rate becomes.

Fig. 18 shows a relationship between the change in galvanic skin conductance and the sweat rate.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A device (100) for estimating an analyte concentration in sweat as released by a sweat gland, the device comprising:
- a measurement input unit (2) configured to obtain at least one of flow rate, pH value and rate of change of pH value (8) of sweat on a user's skin (48),
- a sensor input unit (4) configured to obtain the analyte concentration (10) in the sweat on the user's skin (48), and
- a processing unit (6) configured to correct the analyte concentration (10) according to a correction model (46) for estimating the analyte concentration as released by the sweat gland based on at least one of the flow rate, the pH value and the rate of change of the pH value (8) of the sweat.

2. The device (100) as claimed in claim 1,
wherein the processing unit (6) is configured to correct the sensed analyte concentration (10)
if at least one of the following conditions are met:
the flow rate is below a first flow rate threshold, the pH value is below a first pH value threshold, the rate of change of the pH value is above a first rate of change threshold.

3. The device (100) as claimed in claim 2,
wherein the first flow rate threshold ranges between 0.5 mg/min/cm² and 1.2 mg/min/cm², particularly between 0.8 mg/min/cm² and 1 mg/min/cm² and/or
wherein the first pH value threshold ranges between 2.5 and 6, particularly between 3 and 5, more particularly between 3.5 and 4.5 and/or
wherein the first rate of change threshold ranges between 0.07 per minute and 0.12 per minute, particularly between 0.09 per minute and 0.11 per minute.

4. The device (100) as claimed in any of the preceding claims,
wherein the correction model (46) comprises any of a look-up table, a graph and a mathematic function.

5. The device (100) as claimed in any of the preceding claims,
further comprising a measuring unit (14) configured to measure at least one of the flow rate, the pH value and the rate of change of the pH value (8) of sweat on the user's skin (48).

6. The device (100) as claimed in any of the preceding claims,
further comprising a sensor unit (16) configured to sense the analyte concentration (10) in the sweat on the user's skin (18).

7. The device (100) as claimed in claim 5,
further comprising a fluid switch (20) configured to direct the sweat away from the sensor unit (16) if at least one of the following conditions are met:
the flow rate is below a second flow rate threshold, the pH value is below a second pH value threshold, the rate of change of the pH value is above a second rate of change threshold.

8. The device (100) as claimed in claims 6 to 7,
further comprising a trigger unit (22) configured to power the sensor unit (16) and/or to trigger the fluid switch (20) according to an exposure of the trigger unit (22) to the sweat.

9. The device (100) as claimed in claim 8,
wherein the trigger unit (22) comprises a swelling element (50) configured to swell according to an exposure to sweat, wherein the sensor unit (16) is powered if a size of the swelling element (50) is above a first power threshold and/or wherein the fluid switch (20) is triggered if a size of the swelling element (50) is above a first trigger threshold.

10. The device (100) as claimed in claims 5 to 9,
wherein the measuring unit (14) comprises any of a galvanic skin conductance sensor, skin impedance sensor, CL- sensor, lactate sensor and electromechanical sensor and/or wherein the sensor unit (16) comprises any of an electrochemical sensor, a coloration based sensor, an electrical impedance sensor and a sensor based on labeled antibodies.

11. A switching sensor device (300) for sensing an analyte concentration in sweat as released by a sweat gland, the switching sensor device (300) comprising:
- a sensor unit (16) configured to sense on a user's skin (48) the analyte concentration in the sweat as exposed to the sensor unit (16), and
- a fluid switch (20) configured to direct the sweat on the user's skin (48) away from the sensor unit (16) if at least one of the following conditions are met:
the flow rate is below a fifth flow rate threshold, the pH value is below a fifth pH value threshold, the rate of change of the pH value is above a fifth rate of change threshold.

12. A trigger sensor device (400) for sensing an analyte concentration in sweat as released by a sweat gland, the trigger sensor device (400) comprising:
- a sensor unit (16) configured to sense on a user's skin (48) the analyte concentration in the sweat as exposed to the sensor unit (16), and
- a trigger unit (22) configured to power the sensor unit (16) according to an exposure of the trigger unit (16) to the sweat.

13. A system (200) for estimating an analyte concentration in sweat as released by a sweat gland, the system comprising:
- a measuring unit (14) configured to measure at least one of flow rate, pH value and rate of change of pH value (8) of sweat on a user's skin (48),
- a sensor unit (16) configured to sense the analyte concentration in the sweat on the user's skin (48) as exposed to the sensor unit (16), and
- a device (100) for estimating an analyte concentration in sweat as released by a sweat gland as claimed in claim 1.

14. A method for estimating an analyte concentration in sweat as released by a sweat gland, the method comprising:
- obtaining at least one of flow rate, pH value and rate of change of pH value (8) of sweat on a user's skin,
- obtaining the analyte concentration in the sweat on the user's skin, and
- correcting the sensed analyte concentration according to a correction model for estimating the analyte concentration as released by the sweat gland based on at least one of the flow rate, the pH value and the rate of change of the pH value (8) of the sweat.

15. A computer program comprising program code means for causing a computer to carry out the steps of the method as claimed in claim 14 when said computer program is carried out on the computer.
